# EUROPÄISCHE PATENTSCHRIFT

(11) **EP 1 064 149 B1**
(45) Veröffentlichungstag und Bekanntmachung des Hinweises auf die Patenterteilung: **02.07.2008**
(21) Anmeldenummer: 99915672.2
(22) Anmeldetag: 19.03.1999
(51) Int. Cl.: B32B 27/12, B32B 27/32, A61L 15/24, A61F 13/02

(54) **LAMINAT, VERFAHREN ZU DESSEN HERSTELLUNG SOWIE VERWENDUNG EINES LAMINATS INSBESONDERE ALS MEDIZINISCHES TRÄGERMATERIAL**
LAMINATE, METHOD FOR PRODUCING SAME AND USE OF A LAMINATE ESPECIALLY AS A MEDICINAL SUPPORT MATERIAL
STRATIFIE, SON PROCEDE DE PRODUCTION ET UTILISATION D'UN LAMINE, NOTAMMENT COMME EXCIPIENT MEDICINAL

(30) Priorität: 20.03.1998 DE 19812403; 13.06.1998 DE 19826455
(43) Veröffentlichungstag der Anmeldung: 03.01.2001
(73) Patentinhaber: Beiersdorf Aktiengesellschaft, 20245 Hamburg (DE)
(72) Erfinder: GILLET, Michel, B-4340 Awans (BE); LENTZ, Christoph, B-4890 Froidthier (BE); LENZ, Dirk, D-20253 Hamburg (DE); LINDER, Helmut, D-22459 Hamburg (DE); TIMM, Jürgen, D-21218 Seevetal (DE)
(86) Internationale Anmeldenummer: PCT/EP1999/001849
(87) Internationale Veröffentlichungsnummer: WO 1999/048684

(56) Entgegenhaltungen:
- EP-A- 0 446 431
- WO-A-95/04654
- WO-A-97/23249
- WO-A-97/24222
- WO-A-97/42922
- GB-A- 2 252 528

## Beschreibung

Die Erfindung bezieht sich auf ein Laminat, Verfahren zu dessen Herstellung und die Verwendung im Hygiene- und medical-Bereich, insbesondere als Trägermaterial für Wundschnellverbände und Rollenpflaster und andere Anwendungen, bei denen die elastischen Eigenschaften des Laminats und ein hervorragender Verbund von Vorteil sind.

Laminate, die als medizinischer Träger Verwendung finden, sind vorbeschrieben.

So offenbart die EP 0 446 431 ein Trägermaterial für medizinische Pflaster, das von einem Laminat gebildet wird, das seinerseits besteht aus einer ersten polymeren Filmschicht, einer zweiten, auf der ersten polymeren Filmschicht erzeugten Filmschicht und einer dritten, in der zweiten Schicht teilweise eingebetteten und auf diese Weise darin verankerten Schicht aus einem makroporösen textilen Material. Die erstgenannten Schichten bestehen vorzugsweise aus Polyurethan, das textile Material wird insbesondere gebildet von einem Polyestervlies oder einem Gittertüll. Dann kann das Trägermaterial mit einer selbstklebenden, hautverträglichen Beschichtung versehen sein, vorzugsweise auf der textilen Materialseite.

Aus der WO 97/42922 ist ein Herstellungsverfahren für ein Laminat bekannt, das einseitig mit einer selbstklebenden Beschichtung versehen ist. Dazu wird ein polymerer Film auf einem thermoplastischen Gewebe oder Vlies durch Hitzeeinwirkung aufgeschmolzen, wobei die Hitze nicht vollflächig einwirkt. Auf die Gewebe- oder Vliesseite wird des weiteren eine selbstklebende Beschichtung aufgetragen, auf die wiederum eine Wundauflage aufgelegt werden kann.

Nachteilig bei den bekannten Laminaten ist oft, daß die Laminate bei mechanischen Beanspruchungen delaminieren, was insbesondere bei medizinischen Produkten der Fall ist. Möglich ist weiterhin, daß die Laminate kein sortenreines Produkt darstellen und somit anfallende Produktionsrückstände oder großflächige benutzte Produkte nicht recycelt werden können.

Aufgabe der Erfindung ist es daher, ein Laminat zur Verfügung zu stellen, das die aus dem Stand der Technik bekannten Nachteile vermeidet. Es soll preisgünstig herstellbar und ökologisch unbedenklich sein, auch soll es in der Anwendung einen angenehmen Tragekomfort bieten.

Gelöst wird diese Aufgabe durch ein Laminat, wie es in Anspruch 1 dargelegt ist. Gegenstand der Unteransprüche sind dabei vorteilhafte Weiterbildungen des Laminats, Verfahren zur Herstellung und die Verwendung desselben insbesondere als medizinisches Produkt.

Erfindungsgemäß wird ein Laminat vorgeschlagen, das aus zumindest einer ersten Schicht aus einem elastischen Polymerfilm und einer zweiten Schicht aus einem elastischen Vlies besteht, wobei das fertige Laminat mikro- und/oder makrogeprägt ist und die erste und zweite Schicht vollflächig verbunden sind, wobei Fasern des Vlies vom Polymerfilm umschlossen werden.

Als elastischer Polymerfilm werden insbesondere verwendet Homopolymere aus Polyethylen, Copolymere aus Ethylen und einem α-Olefin mit einer Kohlenstoffanzahl von C₄ bis C₁₀, beispielsweise LDPE, LLDPE, VLLDPE oder ULLDPE oder Polyethylen, hergestellt in einem metallocen-katalysierten Verfahren oder einem "single-site-Typ"-katalysierten Verfahren, Copolymere aus EVA, Ethylen-Alkyl-acrylat, Ethylen-Methyl-acrylat, Ethylen-Acrylsäure und Ionomere, dann Homopolymere und Copolymere von Polypropylen wie isotaktisches, ataktisches und/oder syndiotaktisches PP, Copolymere von PP und PE, Copolymere von PP und Buten und weitere, alle bevorzugt hergestellt über Ziegler-Natta-Katalyse oder Metallocen-Katalyse, schließlich auch Mischungen der genannten Polymere.

In einer bevorzugten Ausführungsform besteht die erste Schicht des Laminats aus einem Mehrschichtaufbau aus einem Copolymeren aus Ethylen und polaren Comonomeren oder einer Mischung aus LDPE und einem LLDPE, hergestellt durch ein metallocen-katalysiertes Verfahren (m-PE).

In einer weiteren bevorzugten Ausführungsform ist der Polymerfilm der ersten Schicht ein Copolymer aus Ethylen und einem α-Olefin mit einer Kohlenstoffanzahl von C₄ bis C₁₀, wobei das Polyolefin ein Schmelzindex zwischen 1 und 20 g/(10 min) und eine Dichte von 860 bis 900 kg/m³ aufweist.

In einer weiteren bevorzugten Ausführungsform besteht die erste Schicht des Laminats aus zwei coextrudierten Schichten mit einer Deckschicht und einer Verbindungsschicht, wobei die Verbindungsschicht aus reinen Polyolefin-Plastomeren ohne Zusatz von Additiven und Farbstoffen besteht.

Weiterhin kann der Polymerfilm der ersten Schicht zu wenigstens 65% ein thermoplastisches Elastomer enthalten.

Als Materialien für die Fasern des Vlieses werden ebenfalls die oben genannten Polymere eingesetzt, und zwar auch als Mischung oder als Coextrudate, des weiteren Viskose und dessen Derivate, Polyester oder modifizierte Polyester und Polyamide eingesetzt.

Die Herstellung eines hierfür eingesetzten Vlieses kann nach dem spun-bond-, dem melt-blown-, dem thermo-bonded-, dem wet-laid-, dem carded-Verfahren oder "Weaving-Verfahren" sowie Kombinationen der aufgeführten Verfahren erfolgen.

Weitere Additive, die den Polymermischungen zugesetzt werden, sind zum Beispiel Hitzestabilisatoren, UV-Stabilisatoren, Antistatic-Zusätze, Anti-slip-Zusätze, antimikrobiell oder antifungizide Substanzen.

Variationen der Zusammensetzung der Polymermischungen oder des Herstellprozesses, die für einen Fachmann offensichtlich sind, sind dabei Bestandteil der Erfindung.

Der Polymerfilm der ersten Schicht weist bevorzugt ein Flächengewicht von 15 bis 150 g/m², insbesondere von 35 bis 60 g/m², und/oder das textile Flächengebilde ein Flächengewicht von 25 bis 200 g/m², insbesondere von 30 bis 100 g/m², auf.

Vorzugsweise auf die Seite des Vlieses eine hautverträgliche selbstklebende Beschichtung aufgetragen.

Die folgenden Verfahrensschritte werden insbesondere verwendet, um dieses Laminat herzustellen. Diese Schritte können kontinuierlich oder separat durchgeführt werden, ohne die Eigenschaften des Produktes zu beeinflussen.
Mischungen von Polymeren mit einem Schmelzindex zwischen 1 und 20 g/(10 min) in Form von Pellets oder einem Granulat werden einem oder mehreren Extrudern zugeführt, dort gemischt und aufgeschmolzen und bilden dann einen kontinuierlichen Fluß (Temperatur der Schmelze liegt zwischen 175 °C bis 330 °C).
Bei Mehrschichtextrusion wird der Strom des schmelzflüssigen Polymeren in einem Adapter (Feed block) zusammengeführt und ein mehrschichtiger Schmelzfilm in einer Breitschlitzdüse ausgeformt. Der Schmelzfilm tritt aus der Breitschlitzdüse aus und wird zwischen zwei Zylindern abgekühlt, einer der Zylinder kann eine Gravur tragen, um dem Film eine Prägung zu geben (Kalanderstation). Der Vliesstoff wird zwischen die beiden Zylinder geführt, so daß der Schmelzfilm auf dem geprägten Zylinder zu liegen kommt. Die Temperatur der Kühlwalzen wird dabei zwischen 10 °C und 65 °C gewählt.
Durch die Gravur der Kühlwalzen bzw. der Gravur der Oberflächen der Kühlwalzen erzielt man besondere Eigenschaften der Oberfläche des Laminats: Eine Mikroprägung (10 bis 200 µm) macht die Oberfläche soft und matt, eine Makroprägung (200 bis 3000 µm gibt dem Film eine textile Anmutung. Im Falle der Mehrschichtextrusion mit zwei oder mehr Schichten wird das "Feed block"-, "multimanifolds"- oder "tandem extrusion coating"-Verfahren verwendet.

Analog kann dieser "Cast"-Prozeß auch mit einem vorgefertigten Film aus den vorbeschriebenen Polymeren, der auf einer Seite geprägt sein kann, und einem Vlies durchgeführt werden, wobei die beiden Schichten der Extrusionsanlage zugeführt werden und durch einen schmelzflüssigen Film aus den vorbeschriebenen Polymeren zusammengefügt werden ("Heat Lamination process").

In einer weiterhin bevorzugten Verfahrensvariante werden zumindest zwei Polyolefin-Granulate in jeweils einem Extruder aufgeschmolzen und gleichzeitig in zumindest zwei Lagen auf das Vlies aufgebracht.

Das Laminat kann gegebenenfalls einseitig, und zwar auf der Vliesseite, mit einer selbstklebenden Beschichtung versehen werden, auf die gegebenenfalls eine Wundauflage aufgelegt wird.

Eine physikalische Perforation des mit Haftklebstoff bestrichenen Trägermaterials wird durch eine Mikroperforationsanlage, bevorzugt gebildet von einer Stachelwalze, bei erhöhten Temperaturen 100 °C bis 130 °C erreicht. Daraus resultiert eine Luftdurchlässigkeit des beschichten Trägermaterials.

Es handelt sich bei dem erfindungsgemäß benutzten Laminat um einen Folie-Vlies-Verbund mit extrem hoher Elastizität in Längs- und Querrichtung, guten polsternden Eigenschaften (durch Einsatz des Vlieses) und einer sehr angenehmen "soften" Folienoberfläche durch eine spezielle Prägung. Vorzugsweise weisen dabei Vlies und Folie ähnliche Rückstellkräfte auf, so daß es bei Dehnungen bis deutlich über 100 % zu keiner Delaminierung des Verbundmaterials kommt.

Neben den polsternden Eigenschaften des Verbundmaterials ist bei Anwendung als Pflaster die Aufnahme von Feuchtigkeit (Wasserdampf) des Vliesstoffes auf der hautzugewandten Seite hervorzuheben (Tragekomfort).

Zur Erzielung dieser Eigenschaften werden bei der Herstellung des Laminates unter anderem spezielle metallocen-LLDPE-Typen eingesetzt, die als flächige Materialien in Verbindung mit der besonderen Prägung der Oberfläche die besondere Haptik (weich und anschmiegsam) und Elastizität ermöglichen.
Weitere Vorteile des Einsatzes der metallocen-PE-Materialien:
a) migrationsarm, d. h. keine Wanderung von niedermolekularen Bestandteilen an die Oberfläche, dadurch gute Verankerung von Haftklebemassen, kein Abfall der Oberflächenspannung auch nach längerer Lagerung;
b) kein Zusatz von Stearaten notwendig (BSE);
c) hohe Reinheit des verwendeten Polymers (geringste Spuren des verwendeten Katalysators);
d) Dichte und Polydispersität des eingesetzten Polymers sind in den gewünschten Bereichen einstellbar.

Die besondere Haptik wird durch die Vielzahl der oben genannten. Komponenten und Prozesse beeinflußt, dann durch die Wahl des Vliesstoffes, der PE-Typen des Films (LLDPE; VLDPE), die Prägung der Oberfläche der Folie und des Vlieses sowie der Art der Prozeßführung zur Herstellung des Laminats.

Insbesondere durch den Einsatz einer 50 µm metallocen-PE(VLDPE)-Folie ergibt sich ein besserer Kontakt zwischen den Vliesfasern und dem PE-Film (durch Viskositätseigenschaften der Polymerschmelze) gegenüber herkömmlichen PE-Typen. Die Folge davon ist, daß der Verbund Folie-Vlies auch unter extremer Dehnung nicht delaminiert.

Bei den meisten Laminaten, die für Hygieneanwendungen (Windeln) hergestellt werden, wird die Vliesseite als Außenseite verwendet, und zwar zumeist wegen der wertvolleren Anmutung, ein direkter Nutzen für das Produkt liegt nicht vor. Bei der Verwendung des erfindungsgemäßen Laminats als medizinisches Trägermaterial wird im Produkt (vorzugsweise Pflaster), die Vliesseite als Innenseite verwendet, die beim bevorzugten punkt- oder rasterförmigen Haftklebstoffauftrag die zusätzliche Funktion als Feuchtigkeit-Speicher erhält.
Diese Eigenschaft verbessert das Haftvermögen des Pflasters auf der Haut, denn üblicherweise zeigen Folienpflaster bei höherer Temperatur und Luftfeuchtigkeit ein extrem eingeschränktes Haftvermögen bei längerer Tragedauer (Feuchtigkeit sammelt sich unter dem (dichten) Folienpflaster an und führt zum schnellen Ablösen des gesamten Produkts).
Weiterhin kann das Laminat zur Herstellung von selbstklebenden Bandagen oder ähnlichen Produkten Einsatz finden.

Die besonderen Eigenschaften des Laminats legt auch die Verwendung als Träger für ein Hygieneartikel nahe, insbesondere Windeln oder Inkontinenzprodukte.

Des weiteren eignet sich das Material auch vorteilhaft für OP-Einweg-Abdeckmaterialien und als Einsatzmaterial für Schutzbekleidung.

Das Laminat stellt eine preisgünstige Variante dar, denn es werden preisgünstige Rohstoffe für den im wesentlichen einstufiger Herstellprozeß eingesetzt.
Metallocen-Polyolefine stellen relativ billige Polymere dar im Vergleich zu anderen thermoplastischen Elastomeren.
Der Prozeß der Schmelzextrusion ist ein sehr wirtschaftliches Verfahren, da es nur eine Modifikation eines Cast-Verfahren zur Herstellung einer Polyclefin-Folie darstellt. Der Schmelzfilm wird lediglich auf einem Vlies statt auf einer Kühlwalze abgelegt.
Es ist kein zusätzlicher Prozeßschritt nötig wie zum Beispiel bei der off-line Laminierung von einer zuvor extrudierten und abgekühlten Folie mit einem Vlies durch Schmelzkleber.

Auch ökologische Gesichtspunkte finden bei der Verwendung des erfindungsgemäßen Laminats Berücksichtigung. Das Produkt in seiner bevorzugten Ausführungsform ist aus einem einzigen. Grundstoff gefertigt, nämlich reinen Olefinen. Dies Laminat ist recycling-fähig, weil das Laminat als ein sortenreines Produkt und anfallende Produktionsrückstände oder großflächige benutzte Produkte wiederverwendet werden können.

Im folgenden sollen anhand mehrerer Bilder die besonders vorteilhaften Eigenschaften des Laminats nochmals dargestellt sowie mittels mehrerer Beispiele die Herstellung eines extrusionslaminierten Verbundproduktes verdeutlicht werden, ohne die beschriebene Erfindung für bestimmte Produktzusammensetzungen und Prozeßschritte unnötig einschränken zu wollen.

Es zeigen
- die Figur 1: den Schichtaufbau des Laminats,
- die Figur 2: die Anlage zur Extrusionslaminierung,
- die Figur 3: die strukturierte Oberfläche durch Mikro-/Makroprägung des Laminates in Form einer schematischen Darstellung,
- die Figur 4: die Oberseite des Laminats in einer mikroskopischen Aufnahme und
- die Figur 5: die Unterseite des Laminats in einer mikroskopischen Aufnahme.

### Beispiele

### Beispiel 1

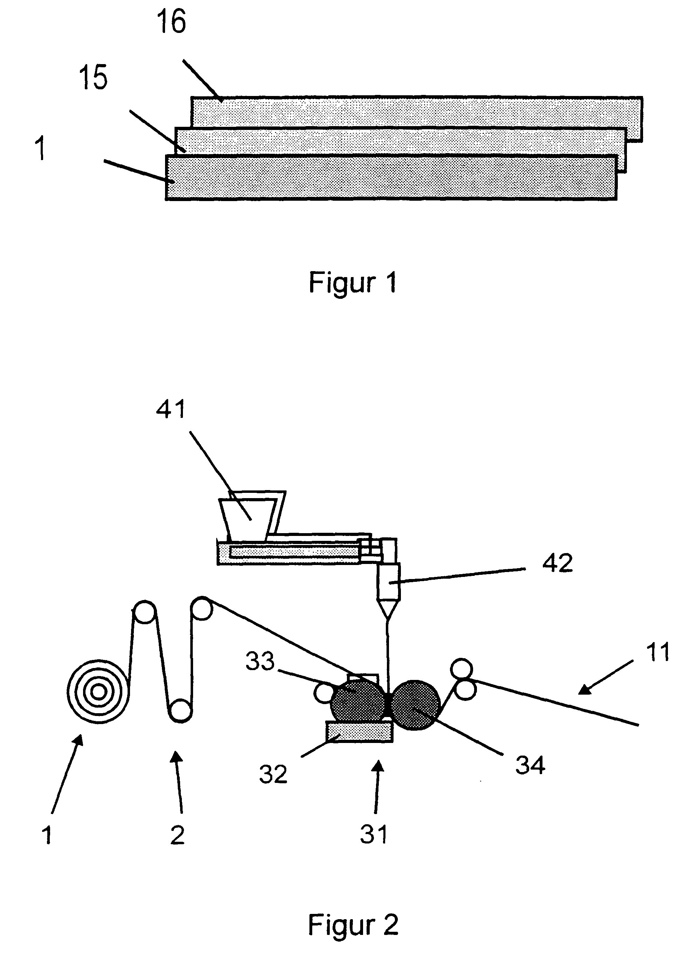

Gemäß Figur 1 setzt sich das Laminat aus drei Schichten zusammen, einer Deckschicht (16), einer Verbindungsschicht (15) und einem Vlies (1).

Die Deckschicht (16) des Laminats besteht aus einer Mischung eines Polyolefin-Plastomers (MI (melt-index) = 3,5 dg/min; Dichte δ = 0,875 g/cm³ (ASTM D-1505)) und einem LDPE (MI = 2,5 dg/min; Dichte δ = 0,916 g/cm³ (ASTM D-1505)) im Verhältnis 90 zu 10. Die Polymermischung wird zusammen mit 5 Gew.-% eines fertigen PE-Farbbatches im Extruder aufgeschmolzen.

Die Verbindungsschicht (15) des Laminats besteht aus einem Polyolefin-Plastomers (MI = 3,5 dg/min; Dichte δ = 0,875 g/cm³ (ASTM D-1505)) und wird in einem zweiten Extruder aufgeschmolzen.

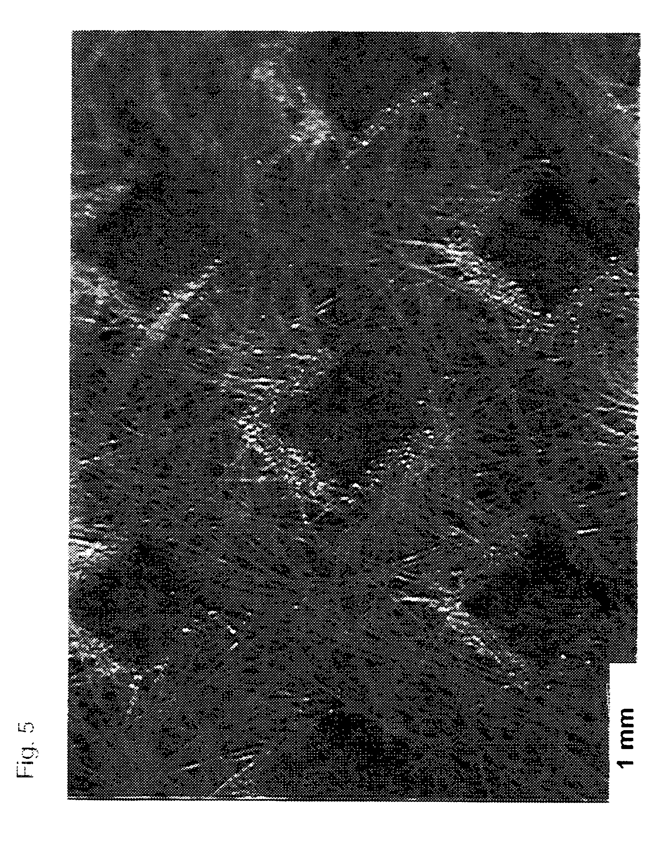

Das Vlies (1) besteht aus einem Spinnvliesstoff aus VLDPE oder einer Mischung aus VLDPE und VLLDPE. Es hat eine rautenförmige Makroprägung, wie sie in Figur 5 veranschaulicht ist (Unterseite des Laminats). Weitere Eigenschaften des Vliesstoffes sind in Tabelle 1 verdeutlicht.

**TABELLE 1: PHYSIKALISCHE EIGENSCHAFTEN DES VLIESSTOFFES**

| **Eigenschaft** | **Methode** | **Meßgröße** |
|---|---|---|
| Flächengewicht | EN 29073 T1 | 50 g/m² |
| Höchstzugkraft längs quer | EN 29073 T1 | 44 N/5 cm 23 N/5 cm |
| Dehnung längs quer | EN 29073 T1 | 194 % 190 % |
| Titer | DIN 53 811 | 3,5 dtex |

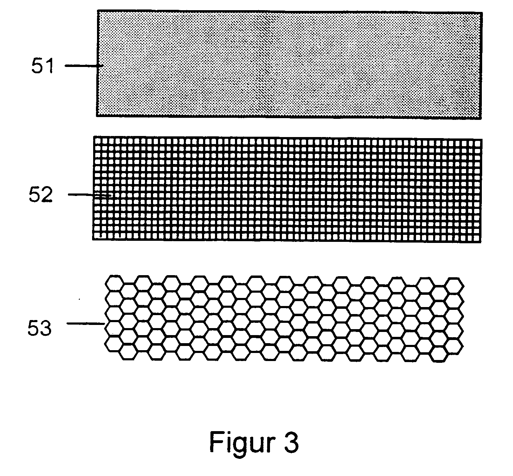

Bei der in Figur 3 dargestellten strukturierten Oberflächen durch Mikro-/Makroprägung des Laminates unterscheidet man:
- Stinflex und Velvaflex (51)
- Taffaflex (52)
- Mayaflex (53)

Die Figur 2 zeigt die Anlage zur Extrusionslaminierung.

Die zwei Polymerschmelzen der Deck- und Verbindungsschicht werden im Feed-block des Extruders (41) zusammengeführt und in der Breitschlitzdüse (42) ausgeformt. Das Verhältnis von Deckschicht (16) zu Verbindungsschicht (15) ist dabei ca. 70 zu 30, das Flächengewicht des Polymerfilms beträgt ca. 50 g/m². Die Temperatur der Breitschlitzdüse beträgt zwischen 240 °C und 260 °C.

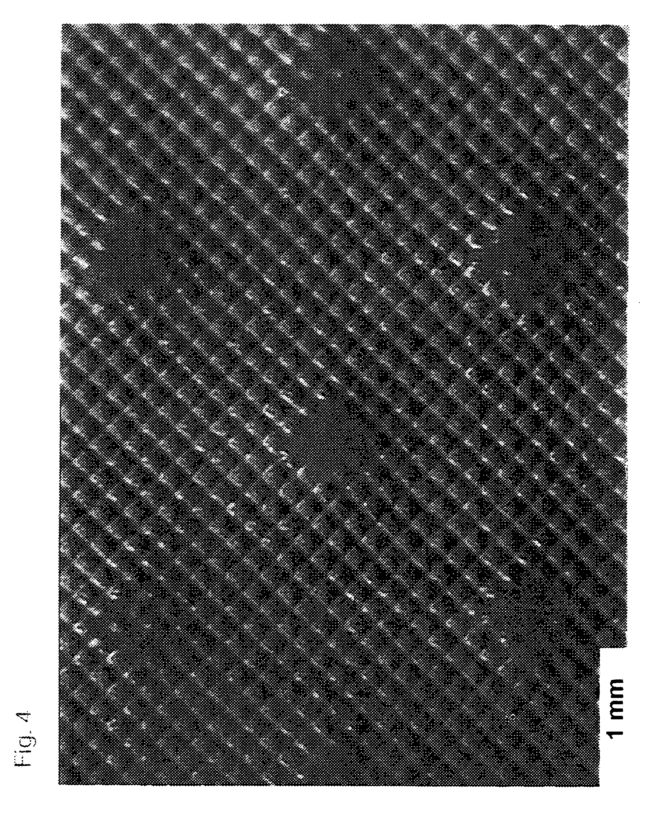

Der zweilagige schmelzflüssige Film wird in der Art auf dem Vlies (1), das über eine Abwickelstation (2) geführt wird, abgelegt, daß die Verbindungsschicht (15) mit dem Vlies (1) in Kontakt kommt und die Deckschicht (16) mit der gekühlten Gravurwalze (34) in der Kalanderstation (31) in Kontakt kommt. Die Deckschicht (16) wird durch den Kontakt mit der Gravurwalze (34) mit einer rautenförmigen Mikroprägung versehen, verdeutlicht in Figur 4 (Oberseite des Laminates). Die vierfach gröbere Makroprägung des Vlieses (1) wird auch im fertigen Laminat (11) reproduziert, so daß von der Folienoberfläche eine überlagerte Mikro-/Makroprägung des gleichen Velvaflex-Musters zu erkennen und zu fühlen ist. Dadurch erhält das Laminat (11) eine wertvollere Anmutung.

Das Laminat (11) wird abschließend aufgewickelt.

Die Dicke des Laminats (11) ist an den Prägepunkten des Vlieses (1) (angeschmolzene Fasern) deutlich geringer. Es entsteht der Eindruck, daß das gesamte Laminat (11) sowohl mikro- als auch makrogeprägt ist. Dadurch ergibt sich ein weicherer textiler Griff des Laminats (11) sowohl auf der Folien- als auch bei der Vliesseite.

Da durch diesen Aufbau die Fasern des Vlieses (1) sehr gut umschlossen werden, erreicht man so einen perfekten Verbund der beiden Komponenten Folie und Vlies. Es kommt auch unter extremer Dehnung zu keiner Delaminierung des Verbundmaterials.

Das beschriebene Composite-Material ist durch weitere Parameter gekennzeichnet (siehe in Tabelle 2).

Das vorliegende Laminat (11) wurde mit Haftklebestoffen vom Acrylat- oder Naturkautschuk-Typ beschichtet. Unter Standard-Konfektionierbedingungen wurden Pflasterstrips (19 mm x 72 mm) ausgestanzt, und es wurde dabei eine Wundauflage aufgelegt.

### Beispiel 2

Die Deckschicht (16) des Laminats besteht aus einem Polyolefin-Plastomer (MI (melt-index) = 3,5 dg/min; Dichte δ = 0,875 g/cm³ (ASTM D-1505)), die zusammen mit einem fertigen PE-Farbbatches (5 Gew.-%) im Extruder aufgeschmolzen wird.

Die Verbindungsschicht (15) des Laminats besteht aus einem Polyolefin-Plastomers (MI = 3,5 dg/min; Dichte δ = 0,875 g/cm³ (ASTM D-1505)), die in einem zweiten Extruder aufgeschmolzen wird.

Es wird der gleiche Vliesstoff aus Beispiel 1 verwendet.

Das Verhältnis von Deckschicht zu Verbindungsschicht ist dabei ca. 60 zu 40, das Flächengewicht des Polymerfilms beträgt ca. 50 g/m².

Die Extrusionsbedingungen sind wie in Beispiel 1 gewählt.
Das beschriebene Composite-Material ist durch weitere Parameter gekennzeichnet (siehe in Tabelle 2).

### Beispiel 3

Die Deckschicht (16) des Laminats besteht aus einer Mischung eines Polyolefin-Plastomers (MI (melt-index) = 3,5 dg/min; Dichte δ = 0,875 g/cm³ (ASTM D-1505)) und einem LDPE (MI = 2,5 dg/min; Dichte δ = 0,916 g/cm³ (ASTM D-1505)) im Verhältnis 80 zu 20. Die Polymermischung wird zusammen mit 5 Gew.-% eines fertigen PE-Farbbatches im Extruder aufgeschmolzen.

Die Verbindungsschicht (15) des Laminats besteht aus einem Polyolefin-Plastomers (MI = 3,5 dg/min; Dichte δ = 0,875 g/cm³ (ASTM D-1505)), die in einem zweiten Extruder aufgeschmolzen wird.

Es wird der gleiche Vliesstoff aus Beispiel 1 verwendet. Das Verhältnis von Deckschicht zu Verbindungsschicht ist dabei ca. 60 zu 40, das Flächengewicht des Polymerfilms beträgt ca. 50 g/m².
Die Extrusionsbedingungen sind wie in Beispiel 1 gewählt.

Das beschriebene Composite-Material ist durch weitere Parameter gekennzeichnet (siehe in Tabelle 2).

### Beispiel 4

Es kommt nur eine Schicht eines PE-Polymers für die Laminierung zum Einsatz.
Die Polymerschicht (16) des Laminats besteht aus einem Polyolefin-Plastomers (MI (melt-index) = 3,5 dg/min; Dichte δ = 0,875 g/cm³ (ASTM D-1505)). Das Polymer wird zusammen mit 4 Gew.-% eines fertigen PE-Farbbatches im Extruder aufgeschmolzen.

Es wird der gleiche Vliesstoff aus Beispiel 1 verwendet.

Das Flächengewicht des Polymerfilms beträgt ca. 50 g/m².

Die Extrusionsbedingungen sind wie in Beispiel 1 gewählt.
Das beschriebene Composite-Material ist durch weitere Parameter gekennzeichnet (siehe in Tabelle 2).

### Beispiel 5: Vergleichsprodukt nicht elastisches Laminat

Die Laminierung des Vliesstoffes aus Beispiel 1 erfolgte hier mit konventionellen LDPE-Typen.

Die Deckschicht (16) des Laminats besteht aus einem LDPE-Polymer (MI (mett-index) = 12 dg/min; Dichte δ = 0,916 g/cm³ (ASTM D-1505)). Das Polymer wird zusammen mit 5 Gew.-% eines fertigen PE-Farbbatches im Extruder aufgeschmolzen.

Die Verbindungsschicht (15) des Laminats besteht aus einem LDPE-Polymer (MI = 12 dg/min; Dichte δ = 0,916 g/cm³ (ASTM D-1505)), die in einem zweiten Extruder aufgeschmolzen wird.

Es wird der gleiche Vliesstoff aus Beispiel 1 verwendet.

Das Verhältnis von Deckschicht zu Verbindungsschicht ist dabei ca. 60 zu 40, das Flächengewicht des Polymerfilms beträgt ca. 60 g/m².

Die Extrusionsbedingungen sind wie folgt: Die Temperaturen des Extruders und der Breitschlitzdüse werden auf 280 °C gesetzt.

Das beschriebene Composite-Material ist durch weitere Parameter gekennzeichnet (siehe in Tabelle 2).

**TABELLE 2: VERGLEICHENDE ÜBERSICHT ÜBER PHYSIKALISCHE DATEN DER HERGESTELLTEN LAMINATE AUS BEISPIEL 1 BIS 5**

| **Parameter / Methode** | **Einheit** | **Beispiel 1** | **Beispiel 2** | **Beispiel 3** | **Beispiel 4** | **Beispiel 5 (Referenz)** |
|---|---|---|---|---|---|---|
| Flächengewicht / DIN 53352 | g/m² | 108,1 | 105,7 | 107,3 | 104,4 | 112,5 |
| Zugkraft längs | N/inch | | | | | |
| Kraft bei 10 % Dehnung | | 11,9 | 11,4 | 11,6 | 9,9 | 18,3 |
| bei 50% Dehnung | | 20,2 | 19,9 | 19,7 | 16,8 | 26,2 |
| bei 100 % Dehnung | | 24,5 | 24,3 | 23,8 | 20,5 | 29,7 |
| Zugkraft quer | N/inch | | | | | |
| Kraft bei 10 % Dehnung | | 6,5 | 6,7 | 6,7 | 6,1 | 14,3 |
| bei 50 % Dehnung | | 12,2 | 12,6 | 13,1 | 11,5 . | 20,5 |
| bei 100 % Dehnung /ASTM D-882 | | 15,1 | 15,5 | 16,4 | 14,4 | -23,0 |
| Permanente Verformung längs *) | % | | | | | |
| bei 50 % Dehnung | | 0 | 0 | 0 | 0 | 0 |
| bei 100 % Dehnung | | 10 | 10 | 16 | 10 | 26 |
| bei 150 % Dehnung | | 40 | 32 | 36 | 36 | 60 |
| Permanente Verformung quer*) | % | | | | | |
| bei 50 % Dehnung | | 0 | 0 | 0 | 0 | 0 |
| bei 100 % Dehnung | | 10 | 10 | 10 | 10 | 27 |
| bei 150 % Dehnung | | 32 | 26 | 30 | 26 | 70 |
| Delaminierung | | keine | keine | keine | keine | leicht |

| | | | | | | |
|---|---|---|---|---|---|---|
| *) Permanente Verformung: Permanente Verformung der Probe in %, die eintritt nach Dehnung um x % der Originallänge. Die Dehnung wird für 30 Sekunden aufrechterhalten und anschließend wird die Probe entlastet. | | | | | | |

### Vergleich der Beispiele.1 bis 5

Die Laminate aus den Beispielen 1 bis 4 zeigen eine geringe plastische Verformung nach 50 bzw. 100%-Dehnung sowohl in Längs- als auch in Querrichtung, was sich bei der Anwendung dieser Laminate als vorteilhaft erweist. Das Vergleichsmaterial (konventionelle PE-Laminat, Beispiel 5) zeigt dagegen eine deutliche höhere plastische Verformung.

Um die Laminate aus den Beispielen 1 bis 4 einer geringen Dehnung zu unterziehen (10 bzw. 50%) sind vergleichsweise geringe Kräfte nötig. Das ist entscheidend für den Komfort beim Endprodukt. Bei dem Vergleichsmaterial aus konventionellem LDPE (Beispiel 5) sind dagegen deutlich höhere Kräfte nötig, um die gleiche Dehnung zu erreichen.

Der Verbund der Laminat-Materialien aus den Beispielen 1 bis 4 läßt sich auch unter starker Dehnung nicht trennen. Das Material aus Beispiel 5 dagegen zeigt bei stärkerer Dehnung leichte Delaminierung.

## Patentansprüche

1. Laminat, bestehend aus zumindest einer ersten Schicht aus einem elastischem Polymerfilm und einer zweiten Schicht aus einem elastischen Vlies, wobei das fertige Laminat mikro- und/oder makrogeprägt ist **dadurch gekennzeichnet, dass** der Polymerfilm auf der Oberfläche eine Mikroprägung aufweist und die erste und zweite Schicht vollflächig verbunden sind, wobei Fasern des Vlieses vom Polymerfilm umschlossen werden.

2. Laminat nach Anspruch 1, **dadurch gekennzeichnet, dass** der Polymerfilm ein Flächengewicht von 15 bis 150g/m², insbesondere von 35 bis 60 g/m², insbesondere von 30 bis 100 g/m², aufweist.

3. Laminat nach den Ansprüchen 1 und 2, **dadurch gekennzeichnet, dass** der Polymerfilm der ersten Schicht aus einem Mehrschichtaufbau aus einem Copolymeren aus Ethylenen und polaren Comonomeren oder einer Mischung aus LDPE und einem LLDPE, hergestellt durch ein metallocen-katalysiertes Verfahren, besteht.

4. Laminat nach einem der Ansprüche 1 bis 3, **dadurch gekennzeichnet, dass** der Polymerfilm der ersten Schicht ein Copolymer aus Ethylen und einem α-Oiefin mit einer Kohlenstoffzahl von C₄ bis C₁₀ ist, wobei das Polyolefin ein Schmelzindex zwischen 1 und 20 g/(10min) und eine Dichte von 860 bis 900 kg/m³ aufweist.

5. Laminat nach den Ansprüchen 1 bis 4, **dadurch gekennzeichnet, dass** die erste Schicht aus zwei coextrudierten Schichten mit einer Deckschicht und einer Verbindungsschicht besteht, wobei die Verbindungsschicht aus reinen Polyolefin-Plastomeren ohne Zusatz von Additven und Farbstoffen besteht.

6. Laminat nach den Ansprüchen 1 bis 5, **dadurch gekennzeichnet, dass** der Polymerfilm der ersten Schicht zu wenigstens 65 % ein thermoplastisches Elastomer enthält.

7. Laminat nach den Ansprüchen 1 bis 6, **dadurch gekennzeichnet, dass** auf die Seite des Vlieses eine selbstklebende Beschichtung aufgetragen ist.

8. Verfahren zur Herstellung eines Laminats gemäß zumindest einem der vorhergehenden Ansprüche, **dadurch gekennzeichnet, dass**
a) ein Polymergranulat oder Mischungen von Polymergranulaten in einem oder mehreren Extrudern aufgeschmolzen werden,
b) die Polymerschmelzen der Extruder in einem Feed-block zusammengeführt werden und ein mehrschichtiger Aufbau des Polymerfilms in der Breitschlitzdüse geformt wird,
c) der gebildete Schmelzfilm auf ein Vlies aufgebracht wird,
d) das so gebildete Laminat durch eine Kalanderstation zusammengepresst und abgekühlt wird,
e) die Oberfläche des Polymerfilms des Laminats geprägt wird, bevorzugt durch eine Stahlzylinderwalze.

9. Verfahren nach Anspruch 8, **dadurch gekennzeichnet, dass** der polymere Schmelzfilm nach der Extrusion zwischen einem vorgefertigten polymeren Film mit elastischen Eigenschaften und dem Vlies geführt wird und anschließend abgekühlt wird.

10. Verwendung eines Laminats nach mindestens einem der vorhergehenden Ansprüche als medizinisches Trägermaterial, wobei auf die Vliesseite eine hautverträgliche selbstklebende Beschichtung aufgetragen ist.

11. Verwendung eines Laminats nach mindestens einem der vorhergehenden Ansprüche als medizinisches Trägermatierial, wobei eine physikalische Perforation des mit der selbstklebenden Beschichtung versehenen Laminats durchgeführt wird.

12. Verwendung eines Laminats nach mindestens einem der vorhergehenden Ansprüche als Träger für ein Hygieneartikel, insbesondere Windeln oder Inkontinenzprodukte.

13. Verwendung eines Laminats nach mindestens einem der vorhergehenden Ansprüche als OP-Einweg-Abdeckmaterialien.

14. Verwendung eines Laminats nach mindestens einem der vorhergehenden Ansprüche als Einsatzmaterial für Schutzbekleidung.

## Claims

1. Laminate composed of at least a first layer of an elastic polymer film and of a second layer of an elastic nonwoven, where the finished laminate has a microscopic and/or macroscopic embossed effect, **characterized in that** the polymer film has a microscopic emboss on the surface and the first and second layers have been bonded over their entire surfaces, where fibres of the nonwoven are enclosed by the polymer film.

2. Laminate according to Claim 1, **characterized in that** the weight per unit area of the polymer film is from 15 to 150 g/m², in particular from 35 to 60 g/m², in particular from 30 to 100 g/m².

3. Laminate according to Claims 1 and 2, **characterized in that** the polymer film of the first layer has a structure of more than one layer of a copolymer of ethylenes and polar comonomers or of a mixture of LDPE and an LLDPE, prepared by a metallocene-catalysed process.

4. Laminate according to one of Claims 1 to 3, **characterized in that** the polymer film of the first layer is a copolymer of ethylene and an α-olefin having a carbon number of from C₄ to C₁₀, where the polyolefin has a melt index of from 1 to 20 g/(10 min) and a density of from 860 to 900 kg/m³.

5. Laminate according to Claims 1 to 4, **characterized in that** the first layer is composed of two coextruded layers with an outer layer and a tie layer, where the tie layer is composed of pure thermoplastic polyolefins without addition of additives or colorants.

6. Laminate according to Claims 1 to 5, **characterized in that** the polymer film of the first layer comprises at least 65% of a thermoplastic elastomer.

7. Laminate according to Claims 1 to 6, **characterized in that** a self-adhesive coating has been applied onto the nonwoven side.

8. Process for producing a laminate according to at least one of the preceding claims, **characterized in that**
a) polymer granules or mixtures of polymer granules are melted in one or more extruders,
b) the polymer melts of the extruders are brought together in a feed block, and a multilayer structure of the polymer film is formed in the slot die,
c) the melt film formed is applied to a nonwoven,
d) the resultant laminate is compressed through a calender unit and cooled, and
e) the surface of the polymer film of the laminate is embossed, preferably using a cylindrical steel roll.

9. Process according to Claim 8, **characterized in that**, after extrusion, the polymer melt film is passed between a previously produced elastic polymer film and the nonwoven, and is then cooled.

10. Use of a laminate according to at least one of the preceding claims as a medical backing material, where a skin-compatible self-adhesive coating has been applied to the nonwoven side.

11. Use of a laminate according to at least one of the preceding claims as a medical backing material, where the laminate provided with the self-adhesive coating is physically perforated.

12. Use of a laminate according to at least one of the preceding claims as carrier for a hygiene item, in particular a nappy or an incontinence product.

13. Use of a laminate according to at least one of the preceding claims as a single-use covering material for uses associated with surgical procedures.

14. Use of a laminate according to at least one of the preceding claims as a material employed in protective clothing.

## Revendications

1. Laminé, constitué par au moins une première couche en un film polymère élastique et une deuxième couche en un non-tissé élastique, le laminé fini étant micromarqué et/ou macromarqué, **caractérisé en ce que** le film polymère présente en sa surface un micromarquage et la première couche et la deuxième couche sont reliées sur toute leur surface, les fibres du non-tissé étant entourées par le film polymère.

2. Laminé selon la revendication 1, **caractérisé en ce que** le film polymère présente un poids surfacique de 15 à 150 g/m², en particulier de 35 à 60 g/m², en particulier de 30 à 100 g/m².

3. Laminé selon les revendications 1 ou 2, **caractérisé en ce que** le film polymère de la première couche est constitué par une structure à plusieurs couches en un copolymère d'éthylène et de comonomères polaires ou d'un mélange de LDPE et d'un LLDPE, préparé par un procédé catalysé par un métallocène.

4. Laminé selon l'une quelconque des revendications 1 à 3, **caractérisé en ce que** le film polymère de la première couche est un copolymère d'éthylène et d'une α-oléfine avec un indice de carbone de C₄ à C₁₀, la polyoléfine présentant un indice de fusion entre 1 et 20 g/(10 min) et une densité de 860 à 900 kg/m³.

5. Laminé selon les revendications 1 à 4, **caractérisé en ce que** la première couche est constituée par deux couches coextrudées avec une couche de recouvrement et une couche d'assemblage, la couche d'assemblage étant constituée de plastomères purs de polyoléfine sans addition d'additifs ni de colorants.

6. Laminé selon les revendications 1 à 5, **caractérisé en ce que** le film polymère de la première couche contient, à raison d'au moins 65%, un élastomère thermoplastique.

7. Laminé selon les revendications 1 à 6, **caractérisé en ce qu'**un revêtement autoadhésif est appliqué sur la face du non-tissé.

8. Procédé pour la préparation d'un laminé selon au moins l'une quelconque des revendications précédentes, **caractérisé en ce que**
a) un granulat de polymère ou des mélanges de granulats de polymère est/sont fondu(s) dans une ou plusieurs extrudeuses,
b) les masses fondues de polymère des extrudeuses sont rassemblées dans un bloc d'alimentation et une structure à plusieurs couches du film polymère est formée dans une filière à fente large,
c) le film de masse fondue formé est appliqué sur un non-tissé,
d) le laminé ainsi formé est pressé dans un poste de calandrage et refroidi,
e) la surface du film polymère du laminé est marquée, de préférence par un cylindre en acier.

9. Procédé selon la revendication 8, **caractérisé en ce que** le film de masse fondue polymère est guidé après l'extrusion entre un film polymère réalisé au préalable avec des propriétés élastiques et le non-tissé et est ensuite refroidi.

10. Utilisation d'un laminé selon au moins l'une quelconque des revendications précédentes comme matériau support médical, un revêtement autoadhésif toléré par la peau étant appliqué sur la face du non-tissé.

11. Utilisation d'un laminé selon au moins l'une quelconque des revendications précédentes comme matériau support médical, une perforation physique du laminé pourvu du revêtement autoadhésif étant réalisée.

12. Utilisation d'un laminé selon au moins l'une quelconque des revendications précédentes comme support pour un article hygiénique, en particulier des langes ou des produits d'incontinence.

13. Utilisation d'un laminé selon au moins l'une quelconque des revendications précédentes comme matériau de recouvrement à usage unique pour salle d'opération.

14. Utilisation d'un laminé selon au moins l'une quelconque des revendications précédentes comme matériau pour vêtements de protection.
